# EUROPEAN PATENT APPLICATION

(11) **EP 4 834 260 A1**
(43) Date of publication of application: **04.11.2026**
(21) Application number: 25867327.6
(22) Date of filing: 01.07.2025
(51) Int. Cl.: A61B 17/94, A61B 17/34, A61M 13/00, A61B 90/00

(54) **PRESSURE MEASUREMENT ABNORMALITY CONTROL METHOD, SYSTEM, AND APPARATUS FOR INSUFFLATOR, AND INSUFFLATOR**

(30) Priority: 21.02.2025 CN 202510192949
(71) Applicant: Microcure (Suzhou) Medical Technology Co., Ltd., Suzhou, Jiangsu 215000 (CN)
(72) Inventor: LIU, Yuxi, Suzhou, Jiangsu 215000 (CN); HE, Xusheng, Suzhou, Jiangsu 215000 (CN); ZHU, Mingshan, Suzhou, Jiangsu 215000 (CN)
(74) Representative: Vogelbruch, Keang
(86) International application number: PCT/CN2025/106523
(87) International publication number: WO 2026/174706

(57) **Abstract**

The present invention relates to the technical field of medical devices, and provides a method for controlling abnormal pressure measurement in an insufflator, including: acquiring an intake pressure, a measurement pressure, and an exhaust pressure; calculating a difference between the intake pressure and the measurement pressure, and a difference between the measurement pressure and the exhaust pressure; when the difference between the intake pressure and the measurement pressure is greater than a first preset difference and less than a second preset difference, and the difference between the measurement pressure and the exhaust pressure is greater than a third preset difference and less than a fourth preset difference, selecting the measurement pressure as a display pressure; otherwise, starting a first abnormal pressure measurement detection procedure. By monitoring the difference between the intake pressure and the measurement pressure, as well as the change in the difference between the measurement pressure and the exhaust pressure, abnormal pressure monitoring caused by equipment failure during a surgery can be identified, thereby increasing equipment stability and reducing surgical risk.

## Description

### CROSS REFERENCE TO RELATED APPLICATION

The present invention claims the priority to the Chinese patent application with the filing No. 2025101929494 filed with the Chinese Patent Office on February 21, 2025, and entitled "METHOD, SYSTEM, AND APPARATUS FOR CONTROLLING ABNORMAL PRESSURE MEASUREMENT IN INSUFFLATOR, AND INSUFFLATOR", which is incorporated herein by reference in entirety.

### TECHNICAL FIELD

The present invention relates to the technical field of medical devices, in particular to a method, system, apparatus for controlling abnormal pressure measurement in an insufflator, and an insufflator.

### BACKGROUND ART

An existing circulating insufflator includes an intake passage, an exhaust passage, and a pressure-measurement passage. The intake passage is configured to provide clean carbon dioxide into the pneumoperitoneum, the exhaust passage is configured to discharge smoke particles from the pneumoperitoneum, and the pressure-measurement passage equipped with a pressure sensor is configured to measure the actual pressure in the pneumoperitoneum. All the three passages need to be connected to a filter to ensure the cleanliness of the circulating gas. However, there is a problem with the above insufflator in that it cannot effectively identify abnormal pressure monitoring caused by equipment failure during a surgery.

For example, a pressure abnormality may be caused by air leakage in a pipeline. Since the filter is a disposable consumable, the three passages need to be manually connected to a new filter for each surgery, which inevitably leads to the problem of inadequate pipeline sealing. If the pipeline of the pressure-measurement passage is not tightly sealed and air leakage occurs, it will lead to abnormal pressure measurement. That is, the pressure value monitored by the pressure-measurement passage would not match the actual pressure value in the pneumoperitoneum.

For example, a pressure abnormality may be caused by pipeline bending or blockage. If the intake passage is blocked, the pressure-measurement pipeline can only monitor that the pressure in the pneumoperitoneum is decreasing. However, the reasons for the pressure decrease in the pneumoperitoneum are multifaceted, and more judgment information cannot be provided, let alone confirming whether the pressure abnormality is caused by the equipment itself.

Since the insufflator adjusts the air intake or deflation based on the pressure value monitored by the pressure-measurement passage to ensure that the actual pressure in the pneumoperitoneum is maintained within an expected pressure range, if air leakage occurs and the pressure value monitored by the pressure-measurement passage does not match the actual pressure value in the pneumoperitoneum, it will pose a significant surgical risk. If bending or blockage occurs but is not detected, the equipment may continuously adjust to maintain the pressure within the expected pressure range, resulting in equipment wear and increasing the instability of the pneumoperitoneum.

Therefore, a new insufflator is now needed, which can better identify abnormal pressure monitoring caused by equipment failure during a surgery, increase equipment stability, and reduce surgical risk.

### SUMMARY

An objective of the present invention is to provide a method, system, apparatus for controlling abnormal pressure measurement in an insufflator, and an insufflator, so as to solve the technical problem in the prior art that abnormal pressure monitoring caused by equipment failure during a surgery cannot be identified.

In a first aspect, an embodiment of the present invention provides a method for controlling abnormal pressure measurement in an insufflator, including steps of: acquiring an intake pressure, a measurement pressure, and an exhaust pressure; calculating a difference between the intake pressure and the measurement pressure, and a difference between the measurement pressure and the exhaust pressure; in response to that the difference between the intake pressure and the measurement pressure is greater than a first preset difference and less than a second preset difference, and the difference between the measurement pressure and the exhaust pressure is greater than a third preset difference and less than a fourth preset difference, selecting the measurement pressure as a display pressure; otherwise, starting a first abnormal pressure measurement detection procedure.

Optionally, the first abnormal pressure measurement detection procedure includes steps of:
in response to that the difference between the intake pressure and the measurement pressure is less than or equal to the first preset difference, and the difference between the measurement pressure and the exhaust pressure is greater than the third preset difference and less than the fourth preset difference, selecting the measurement pressure as the display pressure, and issuing a first warning signal;
in response to that the difference between the intake pressure and the measurement pressure is less than or equal to the first preset difference, and the difference between the measurement pressure and the exhaust pressure is greater than or equal to the fourth preset difference, selecting a static pressure of an exhaust passage as the display pressure, and issuing a second warning signal;
in response to that the difference between the intake pressure and the measurement pressure is greater than or equal to the second preset difference, and the difference between the measurement pressure and the exhaust pressure is greater than the third preset difference and less than the fourth preset difference, selecting the measurement pressure as the display pressure, and issuing a third warning signal;
in response to that the difference between the intake pressure and the measurement pressure is greater than or equal to the second preset difference, and the difference between the measurement pressure and the exhaust pressure is less than or equal to the third preset difference, selecting a static pressure of an intake passage as the display pressure, and issuing a fourth warning signal;
in response to that the difference between the measurement pressure and the exhaust pressure is less than or equal to the third preset difference, and the difference between the intake pressure and the measurement pressure is greater than the first preset difference and less than the second preset difference, selecting the measurement pressure as the display pressure, and issuing a fifth warning signal;
in response to that the difference between the measurement pressure and the exhaust pressure is greater than or equal to the fourth preset difference, and the difference between the intake pressure and the measurement pressure is greater than the first preset difference and less than the second preset difference, selecting the measurement pressure as the display pressure, and issuing a sixth warning signal.

Optionally, the step of selecting a static pressure of an exhaust passage as the display pressure includes: acquiring a pressure of the exhaust passage at a moment after a first preset duration has elapsed from a moment when the exhaust passage stops exhausting.

Optionally, the step of selecting a static pressure of an intake passage as the display pressure includes: acquiring a pressure of the intake passage at a moment after a second preset duration has elapsed from a moment when the intake passage stops intaking.

Optionally, the step of acquiring an intake pressure, a measurement pressure, and an exhaust pressure includes: acquiring a first intake pressure and a second intake pressure, a first measurement pressure and a second measurement pressure, and a first exhaust pressure and a second exhaust pressure; if a difference between the first intake pressure and the second intake pressure, a difference between the first measurement pressure and the second measurement pressure, and a difference between the first exhaust pressure and the second exhaust pressure are all less than or equal to a preset precision difference, then respectively taking an average value of the first intake pressure and the second intake pressure as the intake pressure, an average value of the first measurement pressure and the second measurement pressure as the measurement pressure, and an average value of the first exhaust pressure and the second exhaust pressure as the exhaust pressure; otherwise, starting a first pressure judgment procedure.

Optionally, the first pressure judgment procedure includes:
if the difference between the first intake pressure and the second intake pressure is greater than the preset precision difference, selecting, from the first intake pressure and the second intake pressure, the one whose difference from the measurement pressure is greater than the first preset difference and less than the second preset difference as the intake pressure;
if the difference between the first measurement pressure and the second measurement pressure is greater than the preset precision difference, selecting, from the first measurement pressure and the second measurement pressure, the one whose difference from the intake pressure is greater than the first preset difference and less than the second preset difference, and whose difference from the exhaust pressure is greater than the third preset difference and less than the fourth preset difference, as the measurement pressure; and
if the difference between the first exhaust pressure and the second exhaust pressure is greater than the preset precision difference, selecting, from the first exhaust pressure and the second exhaust pressure, the one whose difference from the measurement pressure is greater than the first preset difference and less than the second preset difference as the exhaust pressure.

In a second aspect, an embodiment of the present invention further provides a system for controlling abnormal pressure measurement in an insufflator, including: a pressure acquisition module, configured to acquire an intake pressure, a measurement pressure, and an exhaust pressure; a difference calculation module, configured to calculate a difference between the intake pressure and the measurement pressure, and a difference between the measurement pressure and the exhaust pressure; and a control module, configured to judge, according to the differences, whether to start a first abnormal pressure measurement detection procedure, and to select a corresponding pressure as a display pressure.

In a third aspect, an embodiment of the present invention further provides an apparatus for controlling abnormal pressure measurement in an insufflator, including a processor, a memory, and a computer program stored in the memory and executable by the processor, wherein the computer program, when executed, causes the processor to implement the steps of the method according to any one of the preceding claims.

In a fourth aspect, an embodiment of the present invention further provides an insufflator, including: the apparatus for controlling abnormal pressure measurement in an insufflator according to the third aspect; an insufflator body; an internal pipeline unit located inside the insufflator body and including an intake passage, a pressure-measurement passage, and an exhaust passage, wherein the intake passage is connected to an intake pump, and the exhaust passage is connected to an exhaust pump; a filter unit connected to the insufflator body, wherein the intake passage, the exhaust passage, and the pressure-measurement passage are all connected to the filter unit; an external pipeline unit including an intake pipeline, a pressure-measurement pipeline, and an exhaust pipeline, wherein the external pipeline unit is connected to the filter unit; a display unit, configured to display a real-time pressure in a pneumoperitoneum; and a pressure sensor unit, including three pressure sensor assemblies, wherein the three pressure sensor assemblies are respectively installed on the intake passage, the exhaust passage, and the pressure-measurement passage, wherein the display unit, the pressure sensor unit, and the internal pipeline unit are all electrically connected to the apparatus for controlling abnormal pressure measurement in an insufflator.

Optionally, each pressure sensor assembly includes two pressure sensors.

The embodiments of the present invention have at least the following technical effects:

The method for controlling abnormal pressure measurement in an insufflator provided by the embodiments of the present invention ensures that the actual abdominal pressure during a surgery remains stable within a suitable range during the normal surgery of the circulating insufflator equipment. Therefore, there is always air intake at the intake end, at which time the intake pressure is greater than the actual abdominal pressure, the measurement pressure is equal to the actual abdominal pressure, and the exhaust end is continuously suctioning, so the exhaust pressure is less than the actual abdominal pressure. Thus, the relationship among the three pressures is: the intake pressure is greater than the measurement pressure, which in turn is greater than the exhaust pressure. If the equipment is normal, and the actual pressure in the pneumoperitoneum fluctuates due to other external factors, such as the doctor's actions or the patient's own changes, then the three pressures fluctuate together. However, the difference between the intake pressure and the measurement pressure, and the difference between the measurement pressure and the exhaust pressure, are still maintained within a reasonable range, i.e., the difference between the intake pressure and the measurement pressure is greater than the first preset difference and less than the second preset difference, and the difference between the measurement pressure and the exhaust pressure is greater than the third preset difference and less than the fourth preset difference. At this time, it indicates that the equipment is normal, and the pressure displayed externally by the equipment for the intra-abdominal pressure may be the measurement pressure. If the difference between the intake pressure and the measurement pressure or the difference between the measurement pressure and the exhaust pressure is not within the above range, it indicates that the equipment itself has malfunctioned. At this time, the first abnormal pressure measurement detection procedure is started to identify the fault type based on the actual changes in the differences, and a pressure value that can represent the actual abdominal pressure is selected as the display pressure. Here, the first preset difference is less than the second preset difference, the third preset difference is less than the fourth preset difference. The first preset difference and the third preset difference may be the same or different, and the second preset difference and the fourth preset difference may be the same or different. This needs to be continuously tested based on the data of the equipment during actual operation to finally obtain reasonable values. By monitoring the difference between the intake pressure and the measurement pressure, as well as the change in the difference between the measurement pressure and the exhaust pressure, the present invention can better identify abnormal pressure monitoring caused by equipment failure during a surgery, thereby increasing equipment stability and reducing surgical risk.

### BRIEF DESCRIPTION OF DRAWINGS

To explain the technical solutions in the embodiments of the present invention or the prior art more clearly, the drawings used in the description of the embodiments or the prior art will be briefly introduced below. Obviously, the drawings in the following description are some embodiments of the present invention. For those of ordinary skill in the art, other drawings may be obtained based on these drawings without creative effort.
FIG. 1 is a schematic flowchart of a method for controlling abnormal pressure measurement in an insufflator provided by an embodiment of the present invention;
FIG. 2 is a schematic flowchart of a first abnormal pressure measurement detection procedure provided by an embodiment of the present invention;
FIG. 3 is a schematic flowchart for acquiring an intake pressure, a measurement pressure, and an exhaust pressure provided by an embodiment of the present invention;
FIG. 4 is a schematic flowchart of a first pressure judgment procedure provided by an embodiment of the present invention; and
FIG. 5 is a structural block diagram of an insufflator provided by an embodiment of the present invention.

### DETAILED DESCRIPTION OF EMBODIMENTS

The technical solutions of the present invention will be described clearly and completely below with reference to the embodiments. Apparently, the described embodiments are only part of the embodiments of the present invention, not all of them. Based on the embodiments of the present invention, all other embodiments obtained by a person of ordinary skill in the art without creative effort shall fall within the protection scope of the present invention.

Those skilled in the art can understand that, unless otherwise defined, all terms (including technical terms and scientific terms) used herein have the same meaning as commonly understood by those of ordinary skill in the art to which the present invention belongs. It should also be understood that terms defined in general dictionaries should be understood to have meanings consistent with their meanings in the context of the prior art, and will not be interpreted in an idealized or overly formal sense unless specifically defined as herein.

Those skilled in the art can understand that the singular forms "a", "an", "the" and "said" used herein may also include plural forms unless specifically stated otherwise. It should be understood that the term "including" used in the specification of the present invention indicates the presence of the stated features, integers, steps, operations, elements and/or components, but does not preclude the presence or addition of one or more other features, integers, steps, operations, elements, components, and/or combinations thereof. The term "and/or" used herein includes any and all combinations of one or more associated listed items.

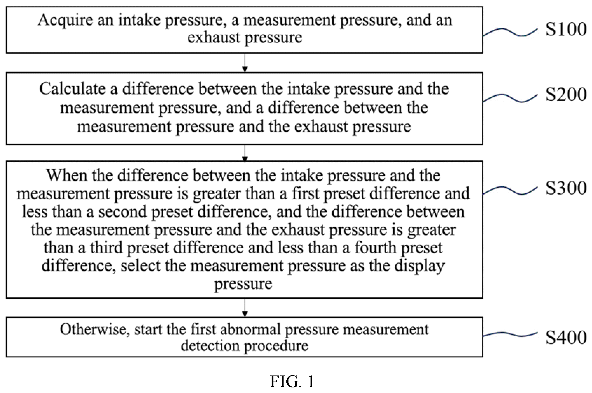

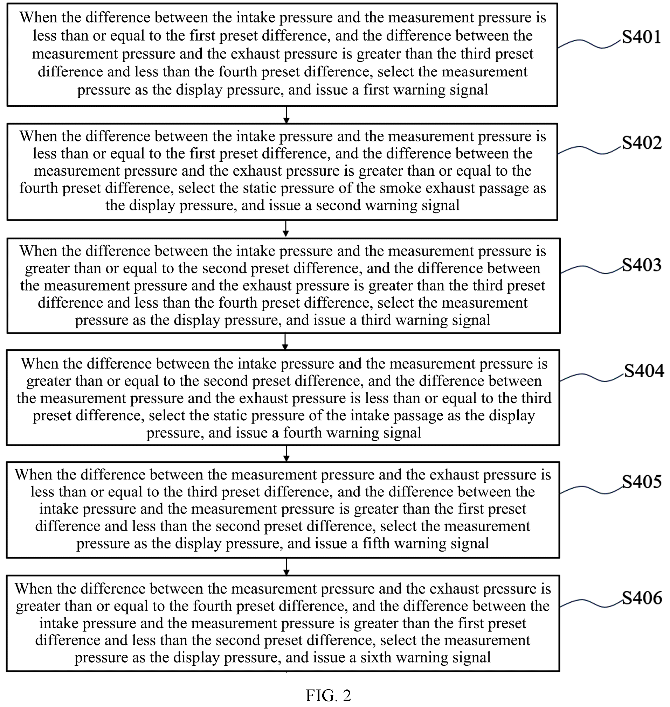

In a first aspect, referring to FIG. 1 to FIG. 4, an embodiment of the present invention provides a method for controlling abnormal pressure measurement in an insufflator, including:
S100: acquiring an intake pressure, a measurement pressure, and an exhaust pressure;
S200: calculating a difference between the intake pressure and the measurement pressure, and a difference between the measurement pressure and the exhaust pressure;
S300: when the difference between the intake pressure and the measurement pressure is greater than a first preset difference and less than a second preset difference, and the difference between the measurement pressure and the exhaust pressure is greater than a third preset difference and less than a fourth preset difference, selecting the measurement pressure as a display pressure;
S400: otherwise, starting a first abnormal pressure measurement detection procedure.

In this embodiment, during the normal operation of the circulating insufflator equipment, the actual abdominal pressure during the surgery is ensured to be stable within a suitable range. Therefore, there is always air intake at the intake end, at which time the intake pressure is greater than the actual abdominal pressure, the measurement pressure is equal to the actual abdominal pressure, and the exhaust end is continuously suctioning, so the exhaust pressure is less than the actual abdominal pressure. Thus, the relationship among the three pressures is: the intake pressure is greater than the measurement pressure, which in turn is greater than the exhaust pressure. If the equipment is normal, and the actual pressure in the pneumoperitoneum fluctuates due to other external factors, such as the doctor's actions or the patient's own changes, then the three pressures fluctuate together. However, the difference between the intake pressure and the measurement pressure, and the difference between the measurement pressure and the exhaust pressure, are still maintained within a reasonable range, i.e., the difference between the intake pressure and the measurement pressure is greater than the first preset difference and less than the second preset difference, and the difference between the measurement pressure and the exhaust pressure is greater than the third preset difference and less than the fourth preset difference. At this time, it indicates that the equipment is normal, and the pressure displayed externally by the equipment for the intra-abdominal pressure may be the measurement pressure. If the difference between the intake pressure and the measurement pressure or the difference between the measurement pressure and the exhaust pressure is not within the above range, it indicates that the equipment itself has malfunctioned. At this time, the first abnormal pressure measurement detection procedure is started to identify the fault type based on the actual changes in the differences, and a pressure value that can represent the actual abdominal pressure is selected as the display pressure. Here, the first preset difference is less than the second preset difference, the third preset difference is less than the fourth preset difference. The first preset difference and the third preset difference may be the same or different, and the second preset difference and the fourth preset difference may be the same or different. This needs to be continuously tested based on the data of the equipment during actual operation to finally obtain reasonable values.

In the embodiment of the present invention, by monitoring the difference between the intake pressure and the measurement pressure, as well as the change in the difference between the measurement pressure and the exhaust pressure, abnormal pressure monitoring caused by equipment failure during a surgery can be better identified, thereby increasing equipment stability and reducing surgical risk.

In this embodiment, step S400 specifically includes the following steps S401 to S406.

S401: when the difference between the intake pressure and the measurement pressure is less than or equal to the first preset difference, and the difference between the measurement pressure and the exhaust pressure is greater than the third preset difference and less than the fourth preset difference, selecting the measurement pressure as the display pressure, and issuing a first warning signal.

In this embodiment, when there is intake air leakage-where intake air leakage here refers to possible air leakage in the part from the location where the pressure sensor is installed to the location where the gas reaches the pneumoperitoneum-the value of the measurement pressure from the pressure-measurement pipeline is the actual pressure of the pneumoperitoneum. Therefore, the measurement pressure is selected as the display pressure. However, the value of the measurement pressure will become lower, and the value of the exhaust pressure will also become lower, but the difference between the measurement pressure and the exhaust pressure remains within the aforementioned range. The value of the intake pressure also becomes lower, causing the difference between the intake pressure and the measurement pressure to be less than or equal to the first preset difference. At this time, a warning signal needs to be issued, for example, the prompt may be the words "Intake Air Leakage", reminding the doctor to check the equipment and eliminate a hidden danger in time.

S402: when the difference between the intake pressure and the measurement pressure is less than or equal to the first preset difference, and the difference between the measurement pressure and the exhaust pressure is greater than or equal to the fourth preset difference, selecting a static pressure of an exhaust passage as the display pressure, and issuing a second warning signal.

In this embodiment, when there is cross-leakage between the intake and the pressure measurement-which means that inside the filter, two independent pipelines that should be tightly sealed are not, and gas that should enter the pneumoperitoneum enters the pressure-measurement pipeline causing cross-leakage-the value of the measurement pressure from the pressure-measurement pipeline is higher than the actual pressure value of the pneumoperitoneum, and the value of the intake pressure becomes lower. Consequently, the difference between the intake pressure and the measurement pressure is less than or equal to the first preset difference, and the difference between the measurement pressure and the exhaust pressure is greater than or equal to the fourth preset difference. Since the measurement pressure can no longer represent the actual pressure of the pneumoperitoneum at this time, the static pressure of the exhaust passage is selected as the display pressure. A warning signal needs to be issued at this time, for example, the prompt may be the words "Cross-leakage between Intake and Pressure Measurement", reminding the doctor to check the equipment and eliminate a hidden danger in time.

S403: when the difference between the intake pressure and the measurement pressure is greater than or equal to the second preset difference, and the difference between the measurement pressure and the exhaust pressure is greater than the third preset difference and less than the fourth preset difference, selecting the measurement pressure as the display pressure, and issuing a third warning signal.

In this embodiment, when there is intake blockage-where intake blockage here refers to possible blockage in the part of the intake passage from the location where the pressure sensor is installed to the location where the gas reaches the pneumoperitoneum-the value of the measurement pressure from the pressure-measurement pipeline is the actual pressure of the pneumoperitoneum. Therefore, the measurement pressure is selected as the display pressure. However, the value of the measurement pressure will become lower, and the value of the exhaust pressure will also become lower, but the difference between the measurement pressure and the exhaust pressure remains within the aforementioned range. Intake blockage causes gas not to reach the pneumoperitoneum, but continuous air intake causes the pressure value in the pipeline to increase, resulting in the difference between the intake pressure and the measurement pressure being greater than or equal to the second preset difference. At this time, a warning signal needs to be issued, for example, the prompt may be the words "Intake Blockage", reminding the doctor to check the equipment and eliminate a hidden danger in time.

S404: when the difference between the intake pressure and the measurement pressure is greater than or equal to the second preset difference, and the difference between the measurement pressure and the exhaust pressure is less than or equal to the third preset difference, selecting a static pressure of an intake passage as the display pressure, and issuing a fourth warning signal.

In this embodiment, when there is pressure measurement leakage or cross-leakage between pressure measurement and exhaust-which refers to possible air leakage in the part of the pressure-measurement passage from the location where the pressure sensor is installed to the location of the pneumoperitoneum, or that inside the filter, two independent pipelines that should be tightly sealed are not, causing the exhaust to have an outward suction effect on the measurement pressure-both lead to the value of the measurement pressure from the pressure-measurement pipeline being lower than the actual pressure value of the pneumoperitoneum, the difference between the measurement pressure and the exhaust pressure being less than or equal to the third preset difference, and the difference between the intake pressure and the measurement pressure being greater than or equal to the second preset difference. Since the measurement pressure can no longer represent the actual pressure of the pneumoperitoneum at this time, the static pressure of the intake passage is selected as the display pressure. A warning signal needs to be issued at this time, for example, the prompt may be the words "Pressure Measurement Leakage", reminding the doctor to check the equipment and eliminate a hidden danger in time.

S405: when the difference between the measurement pressure and the exhaust pressure is less than or equal to the third preset difference, and the difference between the intake pressure and the measurement pressure is greater than the first preset difference and less than the second preset difference, selecting the measurement pressure as the display pressure, and issuing a fifth warning signal.

In this embodiment, when there is exhaust leakage-where exhaust leakage here refers to possible air leakage in the part of the exhaust passage from the location where the pressure sensor is installed to the location where the gas leaves the pneumoperitoneum-the value of the measurement pressure from the pressure-measurement pipeline is the actual pressure of the pneumoperitoneum. Therefore, the measurement pressure is selected as the display pressure. However, the pressure value of the exhaust will increase because the outward suction effect decreases, causing the difference between the measurement pressure and the exhaust pressure to be less than or equal to the third preset difference. However, the difference between the intake pressure and the measurement pressure remains within the aforementioned range. At this time, a warning signal needs to be issued, for example, the prompt may be the words "Exhaust Leakage", reminding the doctor to check the equipment and eliminate a hidden danger in time.

S406: when the difference between the measurement pressure and the exhaust pressure is greater than or equal to the fourth preset difference, and the difference between the intake pressure and the measurement pressure is greater than the first preset difference and less than the second preset difference, selecting the measurement pressure as the display pressure, and issuing a sixth warning signal.

In this embodiment, when there is exhaust blockage-where exhaust blockage here refers to possible blockage in the part of the exhaust passage from the location where the pressure sensor is installed to the location where the gas leaves the pneumoperitoneum-the value of the measurement pressure from the pressure-measurement pipeline is the actual pressure of the pneumoperitoneum. Therefore, the measurement pressure is selected as the display pressure. However, the pressure value of the exhaust will decrease because the outward suction effect increases, causing the difference between the measurement pressure and the exhaust pressure to be greater than or equal to the fourth preset difference. However, the difference between the intake pressure and the measurement pressure remains within the aforementioned range. At this time, a warning signal needs to be issued, for example, the prompt may be the words "Exhaust Blockage", reminding the doctor to check the equipment and eliminate a hidden danger in time.

Specifically, the selecting a static pressure of an exhaust passage as the display pressure includes: acquiring a pressure of the exhaust passage at a moment after a first preset duration has elapsed from a moment when the exhaust passage stops exhausting. In this embodiment, selecting the static pressure of the exhaust passage as the display pressure means that when the exhaust passage is not exhausting, the pressure measured by the pressure sensor on the exhaust passage is the actual pressure in the pneumoperitoneum, and the exhaust pump must be stopped at this time. For example, if the exhaust pressure is selected for display at this time, the exhaust needs to be stopped for 1 second before the pressure value in the exhaust pipeline is acquired. The first preset duration of 1 second here is only for ease of understanding and is not a limitation on its value.

Specifically, the selecting a static pressure of an intake passage as the display pressure includes: acquiring a pressure of the intake passage at a moment after a second preset duration has elapsed from a moment when the intake passage stops intaking. In this embodiment, selecting the static pressure of the intake passage as the display pressure means that when the intake passage is not intaking, the pressure measured by the pressure sensor on the intake passage is the actual pressure in the pneumoperitoneum, and the intake pump must be stopped at this time. For example, if the intake pressure is selected for display at this time, the intake needs to stop for 1 second before acquiring the pressure value in the intake pipeline. The second preset duration of 1 second here is only for ease of understanding and is not a limitation on its value.

In this embodiment, step S100 specifically includes the following steps S401 to S406.

S101: acquiring a first intake pressure and a second intake pressure, a first measurement pressure and a second measurement pressure, and a first exhaust pressure and a second exhaust pressure;
S102: if a difference between the first intake pressure and the second intake pressure, a difference between the first measurement pressure and the second measurement pressure, and a difference between the first exhaust pressure and the second exhaust pressure are all less than or equal to a preset precision difference, then respectively taking an average value of the first intake pressure and the second intake pressure as the intake pressure, an average value of the first measurement pressure and the second measurement pressure as the measurement pressure, and an average value of the first exhaust pressure and the second exhaust pressure as the exhaust pressure;
S103: otherwise, starting a first pressure judgment procedure.

In this embodiment, to ensure that the sensor itself is not faulty, two sensors are provided at the same location to acquire two pressure values. If the difference between the two pressure values is less than or equal to the preset precision difference, it indicates that both pressure values are normal. Therefore, the average value of the two pressure values may be selected as the actual pressure value. The preset precision difference here may be 0.1, 0.01, or other values, selected according to the precision of the sensor itself. If the difference between the two pressure values is greater than the preset precision difference, it indicates that one of the two sensors may be faulty. At this time, it is necessary to start the first pressure judgment procedure to select the correct pressure value.

In this embodiment, step S103 specifically includes the following steps S401 to S406.

S1031: if the difference between the first intake pressure and the second intake pressure is greater than the preset precision difference, selecting, from the first intake pressure and the second intake pressure, the one whose difference from the measurement pressure is greater than the first preset difference and less than the second preset difference as the intake pressure;
S1032: if the difference between the first measurement pressure and the second measurement pressure is greater than the preset precision difference, selecting, from the first measurement pressure and the second measurement pressure, the one whose difference from the intake pressure is greater than the first preset difference and less than the second preset difference, and whose difference from the exhaust pressure is greater than the third preset difference and less than the fourth preset difference, as the measurement pressure;
S1033: if the difference between the first exhaust pressure and the second exhaust pressure is greater than the preset precision difference, selecting, from the first exhaust pressure and the second exhaust pressure, the one whose difference from the measurement pressure is greater than the first preset difference and less than the second preset difference as the exhaust pressure.

In this embodiment, when the difference between two pressure values at the same location is greater than the preset precision difference, the differences that exist among the intake pressure, the measurement pressure, and the exhaust pressure themselves under normal conditions are utilized. The pressure value that conforms to the difference relationship among the intake pressure, the measurement pressure, and the exhaust pressure is the correct pressure value. Doing so will not affect the surgical process due to sensor failure.

The first intake pressure and the second intake pressure are acquired at a same location, the first measurement pressure and the second measurement pressure are acquired at a same location, and the first exhaust pressure and the second exhaust pressure are acquired at a same location.

Taking the difference between the first intake pressure and the second intake pressure as an example, if the difference between the first intake pressure and the second intake pressure is greater than the preset precision difference, and the pressure value of the first intake pressure conforms to the condition that its difference from the measurement pressure is greater than the first preset difference and less than the second preset difference, while the pressure value of the second intake pressure does not conform to this condition, then the pressure value of the first intake pressure should be selected as the intake pressure. The method for acquiring values for other pressures can be derived similarly and will not be repeated here.

Optionally, if one of the first intake pressure and the second intake pressure is selected as the intake pressure, i.e., after the correct pressure value is confirmed, a prompt signal is issued indicating that the other is a fault signal. At this time, the equipment may also issue this prompt signal, giving a prompt that a certain sensor corresponding to the fault signal is faulty, facilitating equipment maintenance after the surgery.

Optionally, if one of the first measurement pressure and the second measurement pressure is selected as the measurement pressure, i.e., after the correct pressure value is confirmed, a prompt signal is issued indicating that the other is a fault signal. At this time, the equipment may also issue this prompt signal, giving a prompt that a certain sensor corresponding to the fault signal is faulty, facilitating equipment maintenance after the surgery.

Optionally, if one of the first exhaust pressure and the second exhaust pressure is selected as the exhaust pressure, i.e., after the correct pressure value is confirmed, a prompt signal is issued indicating that the other is a fault signal. At this time, the equipment may also issue this prompt signal, giving a prompt that a certain sensor corresponding to the fault signal is faulty, facilitating equipment maintenance after the surgery.

Optionally, the above method may be extended to scenarios where one of the two pressure values for the intake pressure, and/or one of the two pressure values for the measurement pressure, and/or one of the two pressure values for the exhaust pressure is problematic, i.e., when sensors at two different locations fail simultaneously or sensors at three different locations fail simultaneously, it can also be detected. The pressure value that conforms to the difference relationship among the intake pressure, the measurement pressure, and the exhaust pressure is the correct pressure value. Therefore, sensors at the three locations can cross-check each other.

In a second aspect, an embodiment of the present invention provides a system for controlling abnormal pressure measurement in an insufflator, including: a pressure acquisition module, configured to acquire an intake pressure, a measurement pressure, and an exhaust pressure; a difference calculation module, configured to calculate a difference between the intake pressure and the measurement pressure, and a difference between the measurement pressure and the exhaust pressure; and a control module, configured to judge, according to the differences, whether to start a first abnormal pressure measurement detection procedure, and to select a corresponding pressure as a display pressure.

In this embodiment, the pressure acquisition module is used to monitor the pressures of the intake passage, the pressure-measurement passage, and the exhaust passage in real time; the difference calculation module is used to calculate the difference between the intake pressure and the measurement pressure, and the difference between the measurement pressure and the exhaust pressure; and the control module is used to judge, according to the differences, whether to start a first abnormal pressure measurement detection procedure, and to select a corresponding pressure as a display pressure. By monitoring the difference between the intake pressure and the measurement pressure, as well as the change in the difference between the measurement pressure and the exhaust pressure, abnormal pressure monitoring caused by equipment failure during a surgery can be better identified, thereby increasing equipment stability and reducing surgical risk.

The functions of the modules in the system for controlling abnormal pressure measurement in an insufflator described in this embodiment are the same as the method steps of the method for controlling abnormal pressure measurement in an insufflator described in the aforementioned embodiments. Therefore, for details not described in this embodiment, please refer to the specific descriptions in the aforementioned embodiments and FIGS. 1 to 4, which will not be repeated here.

In a third aspect, an embodiment of the present invention provides an apparatus for controlling abnormal pressure measurement in an insufflator, including a processor, a memory, and a computer program stored in the memory and executable by the processor, wherein the computer program, when executed, causes the processor to implement the method steps in the method for controlling abnormal pressure measurement in an insufflator described in the aforementioned embodiments.

Through the computer program stored in the memory and executable by the processor, by monitoring the difference between the intake pressure and the measurement pressure, as well as the change in the difference between the measurement pressure and the exhaust pressure, abnormal pressure monitoring caused by equipment failure during a surgery can be better identified, thereby increasing equipment stability and reducing surgical risk.

The method steps implemented when the computer program is executed in the apparatus for controlling abnormal pressure measurement in an insufflator described in this embodiment correspond to the steps of the method for controlling abnormal pressure measurement in an insufflator in the first aspect. For details not described in this embodiment, please refer to the specific descriptions in the aforementioned embodiments and FIGS. 1 to 4, which will not be repeated here.

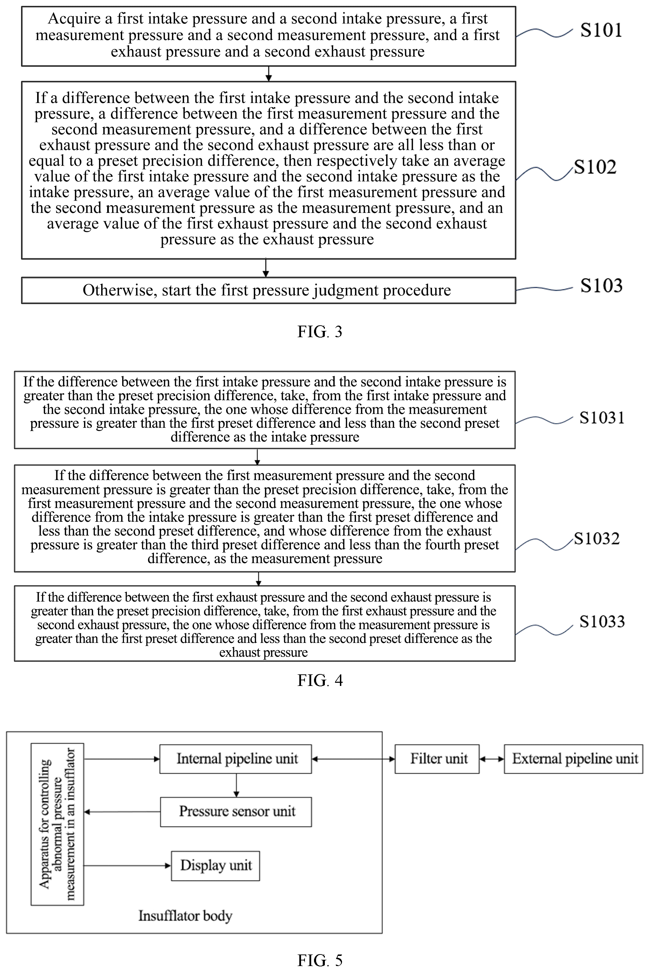

In a fourth aspect, referring to FIG. 5, an embodiment of the present invention provides an insufflator, including: the apparatus for controlling abnormal pressure measurement in an insufflator described in the above embodiments; an insufflator body; an internal pipeline unit located inside the insufflator body and including an intake passage, a pressure-measurement passage, and an exhaust passage, wherein the intake passage is connected to an intake pump and the exhaust passage is connected to an exhaust pump; a filter unit connected to the insufflator body, wherein the intake passage, the exhaust passage, and the pressure-measurement passage are all connected to the filter unit; an external pipeline unit including an intake pipeline, a pressure-measurement pipeline, and an exhaust pipeline, wherein the external pipeline unit is connected to the filter unit; a display unit, configured to display a real-time pressure in a pneumoperitoneum; and a pressure sensor unit including three pressure sensor assemblies, wherein the three pressure sensor assemblies are respectively installed on the intake passage, the exhaust passage, and the pressure-measurement passage; wherein the display unit, the pressure sensor unit, and the internal pipeline unit are all electrically connected to the apparatus for controlling abnormal pressure measurement in an insufflator.

In this embodiment, gas enters the pneumoperitoneum from the insufflator body via the filter unit and the external pipeline unit. The insufflator body includes the internal pipeline unit, the pressure sensor unit, and the apparatus for controlling abnormal pressure measurement in an insufflator. The display unit is also integrated on the insufflator body to provide external information about the pressure in the pneumoperitoneum. The three pressure sensor assemblies are respectively installed on the intake passage, the exhaust passage, and the pressure-measurement passage within the insufflator body. During normal operation of the equipment, the intake pump operates so that there is continuous air intake at the intake end. Therefore, the pressure measured by the sensor on the intake passage, i.e., the intake pressure, is greater than the actual abdominal pressure, while the pressure measured by the sensor on the pressure-measurement pipeline, i.e., the measurement pressure, is equal to the actual abdominal pressure. The exhaust pump operates so that the exhaust end continuously performs suction. Thus, the pressure measured by the sensor on the exhaust pipeline, i.e., the exhaust pressure, is less than the actual abdominal pressure. Thus, the relationship among the three pressures is: the intake pressure is greater than the measurement pressure, which in turn is greater than the exhaust pressure.

If the equipment is normal, the difference between the intake pressure and the measurement pressure, and the difference between the measurement pressure and the exhaust pressure, are maintained within a reasonable range, and the measurement pressure may be used by the equipment for external display of the intra-abdominal pressure. Otherwise, it indicates that the equipment itself has malfunctioned. At this time, the first abnormal pressure measurement detection procedure is started to identify the fault type based on the actual changes in the differences, and a pressure value that can represent the actual abdominal pressure is selected as the display pressure. Generally, blockages are more likely to occur in the external pipeline unit, while air leakage or cross-leakage is more likely to occur at the interfaces of the internal pipeline unit, the filter unit, and the external pipeline unit. Although the filter unit in FIG. 5 is located outside the insufflator body, the meaning here is that the filter unit is disposable and replaceable, which does not mean that the actual filter unit cannot be placed on the insufflator body. If the insufflator body is provided with a structure for placing the filter unit, then the filter unit may be placed on that structure.

In this embodiment, the pressure differences among the three pressure sensors can better identify abnormal pressure monitoring caused by equipment failure during a surgery, thereby increasing equipment stability and reducing surgical risk.

Optionally, if it is necessary to select the pressure measured by the sensor on the intake passage or the pressure measured by the sensor on the exhaust pipeline for display as the intra-abdominal pressure, the apparatus for controlling abnormal pressure measurement in an insufflator sends an instruction to stop the operation of the intake pump or the exhaust pump. Only when the intake pump or the exhaust pump is not in operation can the pressure measured by the sensor on the intake passage or the pressure measured by the sensor on the exhaust pipeline represent the actual pressure in the abdominal cavity.

Optionally, the insufflator may further include an alarm unit, which may issue sounds, lights, text, etc., to remind doctors to eliminate a hidden danger in time.

Optionally, each pressure sensor assembly includes two pressure sensors. In this embodiment, each pressure sensor assembly includes two pressure sensors. This ensures that when one pressure sensor fails, the other pressure sensor can still support normal operation of the equipment without affecting the surgical procedure. Meanwhile, the measured pressure is more reliable, improving equipment stability.

Similarly, for details not described in this embodiment, please refer to the specific descriptions of the aforementioned embodiments and FIGS. 1 to 4, which will not be repeated here.

Those skilled in the art can understand that the steps, measures, and schemes in the various operations, methods, and processes discussed in the present invention may be alternated, modified, combined, or deleted. Optionally, other steps, measures, and schemes in the various operations, methods, and processes already discussed in the present invention may also be alternated, modified, rearranged, decomposed, combined, or deleted. Optionally, steps, measures, and schemes in various operations, methods, and processes disclosed in the present invention that exist in the prior art may also be alternated, modified, rearranged, decomposed, combined, or deleted.

In the description of the present invention, it should be understood that the orientation or locational relationships indicated by terms such as "center", "upper", "lower", "front", "rear", "left", "right", "vertical", "horizontal", "top", "bottom", "inner", "outer", etc., are based on the orientation or locational relationships shown in the drawings. They are used merely for convenience in describing the present invention and simplifying the description, and do not indicate or imply that the referred apparatus or element must have a specific orientation or be constructed and operated in a specific orientation. Therefore, they should not be construed as limiting the present invention.

The terms "first" and "second" are used for descriptive purposes only and cannot be construed as indicating or implying relative importance or implicitly indicating the number of the referred technical features. Thus, features defined by "first" or "second" may explicitly or implicitly include one or more such features. In the description of the present invention, unless otherwise specified, "a plurality of" means two or more.

In the description of the present invention, it should be noted that, unless otherwise explicitly specified and defined, the terms "mount", "connect", and "connection " should be understood broadly. For example, it may be a fixed connection, a detachable connection, or an integral connection; it may be a direct connection, or an indirect connection via an intermediate medium, or may be an internal communication between two elements. For those of ordinary skill in the art, the specific meanings of the above-mentioned terms in the present invention can be understood based on specific circumstances.

In the description of this specification, specific features, structures, materials, or characteristics may be combined in any suitable manner in one or more embodiments or examples.

Finally, it should be noted that the aforementioned embodiments are only used to illustrate the technical solutions of the present invention, and not to limit them. Although the present invention has been described in detail with reference to the foregoing embodiments, those of ordinary skill in the art should understand that they can still modify the technical solutions described in the foregoing embodiments, or perform equivalent substitutions for some or all of the technical features thereof. However, these modifications or substitutions do not cause the essence of the corresponding technical solutions to depart from the scope of the technical solutions of the embodiments of the present invention.

### Industrial Applicability

By monitoring the difference between the intake pressure and the measurement pressure as well as the change in the difference between the measurement pressure and the exhaust pressure, the method for controlling abnormal pressure measurement in an insufflator of the present invention can identify abnormal pressure monitoring caused by equipment failure during a surgery, thereby increasing equipment stability and reducing surgical risk.

## Claims

1. A method for controlling abnormal pressure measurement in an insufflator, comprising steps of:
acquiring an intake pressure, a measurement pressure, and an exhaust pressure;
calculating a difference between the intake pressure and the measurement pressure, and a difference between the measurement pressure and the exhaust pressure;
in response to that the difference between the intake pressure and the measurement pressure is greater than a first preset difference and less than a second preset difference, and the difference between the measurement pressure and the exhaust pressure is greater than a third preset difference and less than a fourth preset difference, selecting the measurement pressure as a display pressure;
otherwise, starting a first abnormal pressure measurement detection procedure.

2. The method for controlling abnormal pressure measurement in an insufflator according to claim 1, wherein the first abnormal pressure measurement detection procedure comprises steps of:
in response to that the difference between the intake pressure and the measurement pressure is less than or equal to the first preset difference, and the difference between the measurement pressure and the exhaust pressure is greater than the third preset difference and less than the fourth preset difference, selecting the measurement pressure as the display pressure, and issuing a first warning signal;
in response to that the difference between the intake pressure and the measurement pressure is less than or equal to the first preset difference, and the difference between the measurement pressure and the exhaust pressure is greater than or equal to the fourth preset difference, selecting a static pressure of an exhaust passage as the display pressure, and issuing a second warning signal;
in response to that the difference between the intake pressure and the measurement pressure is greater than or equal to the second preset difference, and the difference between the measurement pressure and the exhaust pressure is greater than the third preset difference and less than the fourth preset difference, selecting the measurement pressure as the display pressure, and issuing a third warning signal;
in response to that the difference between the intake pressure and the measurement pressure is greater than or equal to the second preset difference, and the difference between the measurement pressure and the exhaust pressure is less than or equal to the third preset difference, selecting a static pressure of an intake passage as the display pressure, and issuing a fourth warning signal;
in response to that the difference between the measurement pressure and the exhaust pressure is less than or equal to the third preset difference, and the difference between the intake pressure and the measurement pressure is greater than the first preset difference and less than the second preset difference, selecting the measurement pressure as the display pressure, and issuing a fifth warning signal; and
in response to that the difference between the measurement pressure and the exhaust pressure is greater than or equal to the fourth preset difference, and the difference between the intake pressure and the measurement pressure is greater than the first preset difference and less than the second preset difference, selecting the measurement pressure as the display pressure, and issuing a sixth warning signal.

3. The method for controlling abnormal pressure measurement in an insufflator according to claim 2, wherein the step of selecting a static pressure of an exhaust passage as the display pressure comprises: acquiring a pressure of the exhaust passage at a moment after a first preset duration has elapsed from a moment when the exhaust passage stops exhausting.

4. The method for controlling abnormal pressure measurement in an insufflator according to claim 2, wherein the step of selecting a static pressure of an intake passage as the display pressure comprises: acquiring a pressure of the intake passage at a moment after a second preset duration has elapsed from a moment when the intake passage stops intaking.

5. The method for controlling abnormal pressure measurement in an insufflator according to claim 1, wherein the step of acquiring an intake pressure, a measurement pressure, and an exhaust pressure comprises:
acquiring a first intake pressure and a second intake pressure, a first measurement pressure and a second measurement pressure, and a first exhaust pressure and a second exhaust pressure;
if a difference between the first intake pressure and the second intake pressure, a difference between the first measurement pressure and the second measurement pressure, and a difference between the first exhaust pressure and the second exhaust pressure are all less than or equal to a preset precision difference, then respectively taking an average value of the first intake pressure and the second intake pressure as the intake pressure, an average value of the first measurement pressure and the second measurement pressure as the measurement pressure, and an average value of the first exhaust pressure and the second exhaust pressure as the exhaust pressure;
otherwise, starting a first pressure judgment procedure.

6. The method for controlling abnormal pressure measurement in an insufflator according to claim 5, wherein the first pressure judgment procedure comprises:
if the difference between the first intake pressure and the second intake pressure is greater than the preset precision difference, selecting, from the first intake pressure and the second intake pressure, the one whose difference from the measurement pressure is greater than the first preset difference and less than the second preset difference as the intake pressure;
if the difference between the first measurement pressure and the second measurement pressure is greater than the preset precision difference, selecting, from the first measurement pressure and the second measurement pressure, the one whose difference from the intake pressure is greater than the first preset difference and less than the second preset difference, and whose difference from the exhaust pressure is greater than the third preset difference and less than the fourth preset difference, as the measurement pressure; and
if the difference between the first exhaust pressure and the second exhaust pressure is greater than the preset precision difference, selecting, from the first exhaust pressure and the second exhaust pressure, the one whose difference from the measurement pressure is greater than the first preset difference and less than the second preset difference as the exhaust pressure.

7. The method for controlling abnormal pressure measurement in an insufflator according to claim 6, wherein the method further comprises:
if one of the first intake pressure and the second intake pressure is selected as the intake pressure, issuing a prompt signal indicating that the other is a fault signal;
if one of the first measurement pressure and the second measurement pressure is selected as the measurement pressure, issuing a prompt signal indicating that the other is a fault signal; and
if one of the first exhaust pressure and the second exhaust pressure is selected as the exhaust pressure, issuing a prompt signal indicating that the other is a fault signal.

8. The method for controlling abnormal pressure measurement in an insufflator according to claim 5, wherein the first intake pressure and the second intake pressure are acquired at a same location, the first measurement pressure and the second measurement pressure are acquired at a same location, and the first exhaust pressure and the second exhaust pressure are acquired at a same location.

9. A system for controlling abnormal pressure measurement in an insufflator, comprising:
a pressure acquisition module, configured to acquire an intake pressure, a measurement pressure, and an exhaust pressure;
a difference calculation module, configured to calculate a difference between the intake pressure and the measurement pressure, and a difference between the measurement pressure and the exhaust pressure; and
a control module, configured to judge, according to the differences, whether to start a first abnormal pressure measurement detection procedure, and to select a corresponding pressure as a display pressure.

10. An apparatus for controlling abnormal pressure measurement in an insufflator, comprising a processor, a memory, and a computer program stored in the memory and executable by the processor, wherein the computer program, when executed, causes the processor to implement the steps of the method according to any one of claims 1 to 8.

11. An insufflator, comprising: the apparatus for controlling abnormal pressure measurement in an insufflator according to claim 10;
an insufflator body;
an internal pipeline unit, located inside the insufflator body and comprising an intake passage, a pressure-measurement passage, and an exhaust passage, wherein the intake passage is connected to an intake pump, and the exhaust passage is connected to an exhaust pump;
a filter unit, connected to the insufflator body, wherein the intake passage, the exhaust passage, and the pressure-measurement passage are all connected to the filter unit;
an external pipeline unit, comprising an intake pipeline, a pressure-measurement pipeline, and an exhaust pipeline, wherein the external pipeline unit is connected to the filter unit;
a display unit, configured to display a real-time pressure in a pneumoperitoneum; and
a pressure sensor unit, comprising three pressure sensor assemblies, wherein the three pressure sensor assemblies are respectively installed on the intake passage, the exhaust passage, and the pressure-measurement passage,
wherein the display unit, the pressure sensor unit, and the internal pipeline unit are all electrically connected to the apparatus for controlling abnormal pressure measurement in an insufflator.

12. The insufflator according to claim 11, wherein each pressure sensor assembly comprises two pressure sensors.

13. The insufflator according to claim 11, further comprising an alarm unit, wherein the alarm unit is electrically connected to the apparatus for controlling abnormal pressure measurement in an insufflator.

14. The insufflator according to claim 12, wherein the display unit is integrated into the insufflator body.
